# EUROPEAN PATENT APPLICATION

(11) **EP 1 477 493 A1**
(43) Date of publication of application: **17.11.2004**
(21) Application number: 03009894.1
(22) Date of filing: 15.05.2003
(51) Int. Cl.: C07H 15/26, C07H 15/203, C07H 15/24, G01N 33/532, C07F 9/38, C07F 9/40, C07F 9/09, C07K 5/062, C07F 9/12, C07K 5/02, C07K 14/71, C07K 7/06

(54) **Isotope coded affinity tags**

(71) Applicant: Bayer HealthCare AG, 51368 Leverkusen (DE)
(72) Inventor: Auriel, Daniel, Dr., 1300 Wavre (BE); Lerchen, Hans-Georg, Dr., 51375 Leverkusen (DE); Immler, Dorian, Dr., 51375 Leverkusen (DE); Wesener, Joachim, Dr., 51061 Köln (DE); Schumacher, Andreas, Dr., 79588 Efringen-Kirchen (DE)

(57) **Abstract**

The invention relates to isotope coded affinity tags for the mass spectrometric analysis of amino acid phosphates, phosphopeptides or phosphoproteins, their use and kits comprising the same.

## Description

The invention relates to isotope coded affinity tags for the mass spectrometric analysis of amino acid phosphates, phosphopeptides or phosphoproteins, their use and kits comprising the same.

The invention provides a general chemical method for the selective labelling of phosphate groups. The method is applicable to the selective labelling of phosphorylated amino acids or peptides.

Proteomics technologies open the possibility to identify new biological targets and markers. To achieve this goal, different methods for the analysis of proteins' expression patterns can be used ((a) S. P. Gygi et al., Proc. Natl. Acad. Sci. U. S. A., 2000, 97, 9390; (b) D. R. Goodlett et al., Proteome Protein Anal., 2000, 3; (c) S. P. Gygi et al., Curr. Opin. Biotechnol., 2000, 11, 396).

One particularly promising methodology involves the detection and analysis of labelled protein fragments by mass spectrometry. The utilization of different isotopically-coded affinity tags (ICATs® = Isotope Coded Affinity Tags) enables a quantitative analysis of complex protein mixtures based the LC-separation and characterization and of labelled peptides with tandem-mass spectrometry ((a) S. P. Gygi et al., Nature Biotechnology, 1999, 17, 994; (b) R. H. Aebersold et al., WO 00/11208). This methodology involves the relative quantification of two or more protein mixtures that are labelled with ICATs carrying different isotopic tags and thus showing a distinct mass difference. The differentially labelled mixtures are then combined, treated under proteolytic conditions and purified by affinity chromatography. The labelled peptide fractions are subsequently analysed by a combination of liquid chromatography and tandem mass spectrometry (LC-MS/MS). Pairs of labelled peptides, distinct from each other in mass but otherwise chemically identical, will co-elute in the chromatography step. The distinct mass difference allows for a relative quantitation; identification of the parent proteins is provided via peptide sequence information from tandem-MS and data base searches.

The state-of-the-art ICATs are able to react with cystein residues, but they do not provide a means for the analysis of post-translational. modifications like phosphorylation, glycosylation, acetylation, prenylation, ubiquitination, etc. that proteins may undergo. Since such post-translational modifications have a direct impact on cellular regulation processes their profiling in protein analysis is especially relevant. Phosphorylation in particular, should be addressed.

A review on current approaches to phosphoproteome analysis is provided by T. Veenstra et al (Biochem. Biophys. Res. Commun. 2002, 290, 885).

In an advanced stage is the enrichment of phosphopeptides by immobilized metal affinity chromatography (IMAC; Anal. Biochem. 1986b, 154, 254).

The current state-of-the-art methodologies for the targeting of phosphoproteins are cumbersome and often limited in applicability. For example Aebersold's technology described in WO 01/96869 and in Nature Biotechnol., 2001, 19, 375 involves 1) the protection of all free nucleophilic basic residues, 2) the formation of unreactive amides and phosphoramides from all available acidic groups, 3) the selective cleavage of the phosphoramides under mild acidic conditions, 4) formation, of new phosphoramides carrying an additional reactive thiol functionality 5) capturing and isolation of the reactive thiols linked to phosphates and 6) cleavage of all protecting groups and amides under strong acidic conditions, in order to allow detection, identification and quantification.

Other approaches include phosphate ß-elimination and subsequent modification of the dehydro amino acid derivatives with appropriately tagged linker molecules (e.g. M.B. Goshe et al, Anal. Chem. 2001, 73(11), 2578). This methodology, however, is limited to phospho serine and phospho threonine residues.

Therefore, the development of reagents allowing direct reaction with phosphate residues in complex mixtures is desirable. Such reagents should ideally be compatible with the direct application of the ICAT technology.

The present invention relates to compounds of the formula 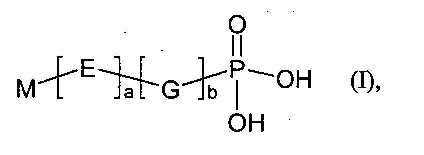 in which
- a and b: each independently are 0 or 1,
- M: is selected from the group consisting of
hydrogen,
phenyl,
an amino protecting group, or
a group A-L,
where
A is the residue of an affinity ligand or a solid-phase support and
L is a covalent linker between A and E in the case of a being 1 or between A and G in the case of a being 0,
the linker L having the formula 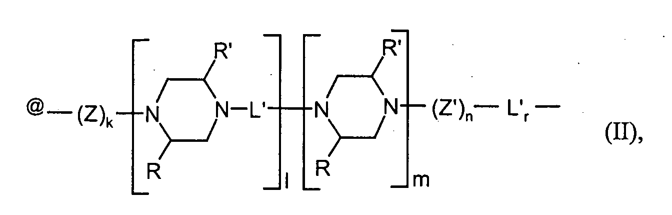 wherein
@ denotes the terminus linked to A,
k, l, m, n, r each independently is 0, 1, 2, 3, 4 or 5, provided that their summation k + 1 + m + n + r amounts to at least 1 and not more than 20,
Z is an amino acid group NH-CHR^{x}-(CH₂)ₓ-CO, where R^{x} is an amino acid side-chain and x is 0, 1, 2, 3, 4 or 5,
L' is a bridge enabling the covalent linkage between two piperazine groups, between two substituted amino groups or between a substituted amino group and a piperazine group,
R, R' are independently selected amino acid side-chains attached to a piperazine ring,
Z' is an amino acid residue CO-(CH₂)_{y}-CHR^{y}-NH, where R^{Y} is an amino acid side-chain and y is 0, 1, 2, 3, 4 or 5, which is distinct from Z due to the different orientation of the terminal CO and NH groups,
- E: is a residue of an amino alcohol moiety of the formula 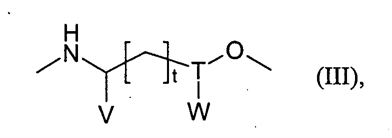 wherein
V is hydrogen, carboxy (-COOH) or an optionally arylsubstituted alkoxycarbonyl,
t is 0, 1, 2, 3, 4 or 5,
W is hydrogen or methyl and
T is CH or p-phenylen with the proviso that in the case of T being p-phenylen t is 1 and W is hydrogen,
- G: is a residue of a glucose derivative of the formula 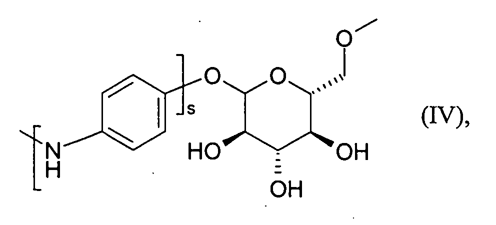 wherein s is 0 or 1 and the configuration at the anomeric carbon atom is α or ß or a mixture of α and ß.
and their salts.

For the purposes of the present invention, the subsituents have the following meanings, unless otherwise specified:
Alkyl per se and "alk" in alkoxy and alkoxycarbonyl represents a linear or branched alkyl radical having generally 1 to 6, preferably 1 to 4 and particularly preferably 1 to 3 carbon atoms, representing illustratively and preferably methyl, ethyl, n-propyl, isopropyl, tert-butyl, n-pentyl and n-hexyl.
Alkoxy illustratively and preferably represents methoxy, ethoxy, n-propoxy, isopropoxy, tert-butoxy, n-pentoxy and n-hexoxy.
Alkoxycarbonyl illustratively and preferably represents methoxycarbonyl, ethoxycarbonyl, n-propoxycarbonyl, isopropoxycarbonyl, tert-butoxycarbonyl, n-pentoxycarbonyl and n-hexoxycarbonyl.
Aryl represents a mono- to tricyclic aromatic carbocyclic radical having generally 6 to 14 carbon atoms, illustratively and preferably representing phenyl (Ph), naphthyl and phenanthrenyl.
An amino protecting group is tert-Butoxycarbonyl (Boc), Fluorenyl-9-methoxycarbonyl (Fmoc), Benzyloxycarbonyl (Z) or another amino protecting group as described e.g. in Houben Weyl; Methoden der Organischen Chemie; Vierte Auflage; Band XV Teil 1 und 2; Georg Thieme Verlag Stuttgart 1974, or in Hans-Dieter Jakubke and Hans Jeschkeit: Aminosäuren, Peptide, Proteine; Verlag Chemie, Weinheim 1982, preferably Boc, Fmoc or Z.
A^{@} symbol next to a bond denotes the point of attachment in the molecule.

If radicals in the compounds according to the invention are substituted, the radicals, unless otherwise specified, can be substituted by one or more identical or different substituents. A substitution with up to three identical or different substituents is preferred. Very particular preference is given to substitution with one substituent.

The approach of this invention relies on the direct and selective formation of pyrophosphates from preactivated pyrophosphates generated in analogy to the dinucleotide formation described previously by Robins et al. (US Patent 4,544,741, 1985).

The organic phosphates of formula (I) are transformed to activated pyrophosphates to enable a selective formation of pyrophosphates from amino acid phosphates, phosphopeptides and phosphoproteins as shown in scheme 1. The modification of the phosphates allows for both, the attachment of capturing molecules such as an affinity ligand or solid phase support and isotope labelling to enable analysis and relative quantification by mass-spectrometry.

The objects of the invention are organic phosphates of formula (I), which are pre-activated and subsequently attached to amino acid phosphates, phosphopeptides or phosphoproteins according to scheme 1,

The organic phosphates of formula (I) are transformed to respective activated pyrophosphate derivatives of formula (V) according to scheme 1. These derivatives of formula (V) selectively react with phosphorylated amino acid residues (VI) where HA is the deprotonated side chain of an hydroxy amino acid like serine, threonine or tyrosine to pyrophosphates (VII) in the presence of other unprotected functional groups. (VI) might be protected with R₁ and R₂ residues of amino and carboxy protecting groups respectively, or unprotected with R₁ = H and R₂ = OH or (VI) might be part of a peptide or a protein with R₁ and R₂ = peptide residues. Therefore, this method allows for both, a selective labelling and a capturing of phosphate groups in phosphopeptides and phosphoproteins.

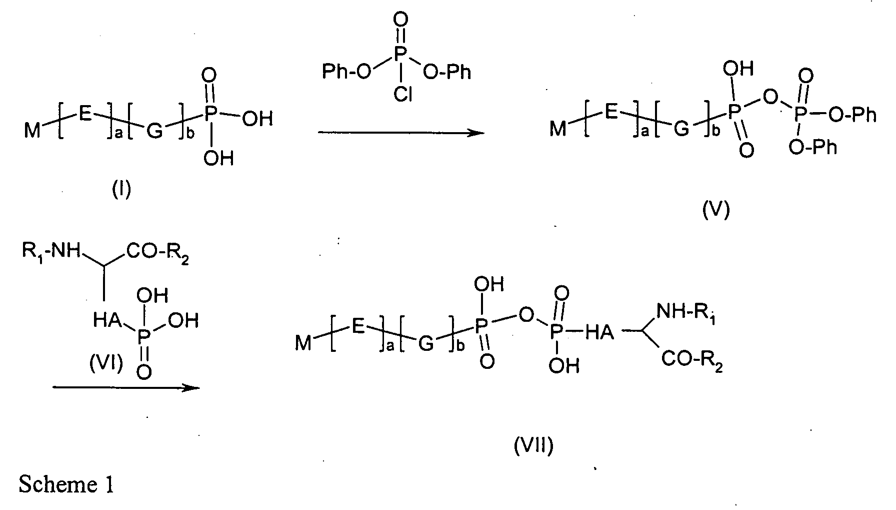

The invention further relates to compounds of formula 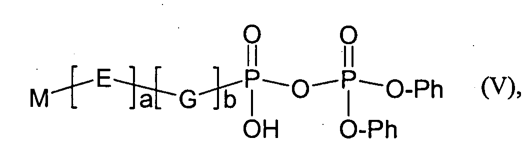 in which
a, b, M, E, G have the same meaning as in formula (I) and
- Ph: represents phenyl.

The invention further relates to compounds of formula 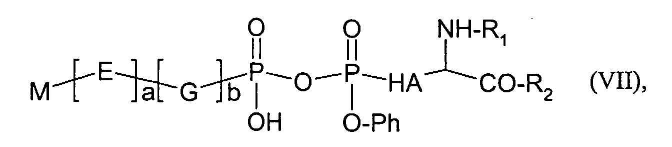 in which
- a, b, M, E, G: have the same meaning as in formula (I),
- Ph: represents phenyl,
- HA: represents a deprotonated side chain of an hydroxy amino acid,
- R₁, R₂: each independently represents a residue of amino and carboxy protecting groups respectively or
are R₁ = H and R₂ = OH or
each independently represents a peptide residue.

Affinity Linkers L of formula (II) without the terminal L'ᵣ group are described in international application PCT/EP02/12105 incorporated herein by reference.

The linker L can exist in different isotopically-coded forms.

In a particular embodiment of the invention the phosphate markers described can carry isotopic labels (deuterium, ¹³C- and ¹⁵N) enabling the relative quantification of two different samples tagged with light and heavy linker molecules by LC-MS. Such tags may be incorporated in G based on Glucose commercially available with 6 ¹³C-labels or in the linker part L based on deuterium-, ¹³C-, and/or ¹⁵N-labelled amino acid, particularly glycine residues.

The affinity ligand A allows the selective enrichement of the samples through affinity chromatography. An example for such a ligand is biotinoyl residue, which enables capturing with avidin or streptavidin columns. A preferred affinity ligand is biotinoyl.

Alternatively, this enrichment can also be achieved by selective immobilisation on an adequate solid phase or resin A. Adequate resins are, for example, hydroxy-functionalised resins with a polyethylenglycol-basis and furthermore, other resins routinely used for solid-phase peptide synthesis such as Trityl resins, benzylalcohol-based Sasrin-resins, benzylalcohol-based Wang-resins, oder TCP (tritylchloride-polystyrol)-resins. Further preferred resins are resins on silica gel basis with functional groups enabling the attachment of the linker part.

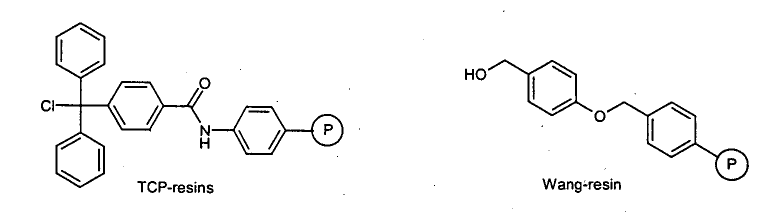

The definitions of radicals detailed in the particular or preferred combinations of radicals are also arbitrarily replaced by radical definitions in a different combination, independently of the particular combinations of radicals indicated.

Very particular preference is given to combinations of two or more of the above-mentioned preference ranges.

The invention further relates to a process for the labeling of amino acid phosphates, phosphopeptides or phosphoproteins comprising
(i) providing one ore more differently isotope labeled compounds of formula (I),
(ii) transforming said one or more compounds to one or more compounds of formula (V) and
(iii) reacting the one ore more compounds of formula (V) with a sample comprising at least one phosphorylated amino acid, peptide or protein derivative.

The invention further relates to the use of one or more differently isotope labeled compounds according to the invention as reagent for the mass spectrometric analysis of amino acid phosphates, phosphopeptides or phosphoproteins.

Preference is given to a use according to the invention for identifying one or more amino acid phosphates, phosphopeptides or phosphoproteins in one or more samples comprising amino acid phosphates, phosphopeptides or phosphoproteins.

Preference is also given to a use according to the invention for determining the relative expressions levels of one or more amino acid phosphates, phosphopeptides or phosphoproteins in one or more samples comprising amino acid phosphates, phosphopeptides or phosphoproteins.

The invention further relates to kits for the mass spectrometric analysis of amino acid phosphates, phosphopeptides or phosphoproteins, comprising as reagents one or more differently isotope labeled compounds according to the invention.

The compounds of formulae (I) to (VII) can be prepared as described herein or similarly to known processes.

### Examples

### Abreviations:

- Boc: - tert-Butoxycarbonyl
- DCM: - Dichlormethane
- DMSO: - Dimethylsulfoxide
- ESI: - Electrospray-Ionisation
- Fmoc: - Fluorenyl-9-methoxycarbonyl
- MS: - Mass spectrometry
- RT: - Room temperature
- TFA: - Trifluoracetic acid
- THF: - Tetrahydrofuran
- TLC: - Thin layer chromatography
- Z: - Benzyloxycarbonyl

### Example series A: Reactive pyrophosphate intermediates

### General formula:

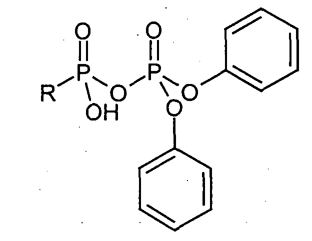

### General procedure:

0,075 mmol of the particular phosphate precursor are dissolved in 25 ml methanol with 32,7 µl tri-n-octylamine and stirred for 15 min. at RT. The solvent is removed *in* *vacuo* and the residue is repeatedly treated with DCM which is also removed, in order to eliminate all traces of water.

After drying under high vacuum overnight, the product is dissolved under anhydrous conditions in 25 ml dry dioxan and 20,156 µl (0,097 mmol) of diphenylchlorophosphate are added, followed by 33,86 µl (0,142 mmol) tri-n-butylamine. After 2 hours of stirring, the solvent is removed *in vacuo* and ether is added to residue to precipitate the product. The ether is then removed by decantation. The compounds are kept under high vacuum until used.

### Example A.1 (in situ)

### Phosphate precursor:

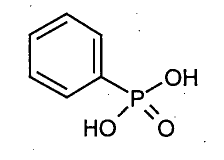

### Reactive intermediate:

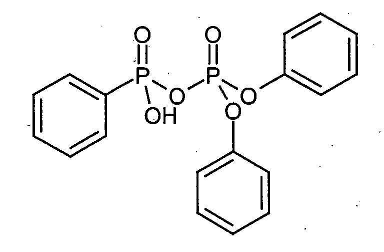

### Example A.2

### Phosphate precursor:

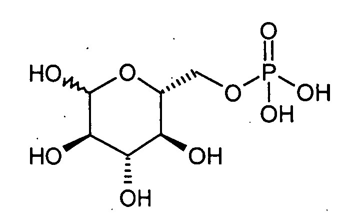

### Reactive intermediate:

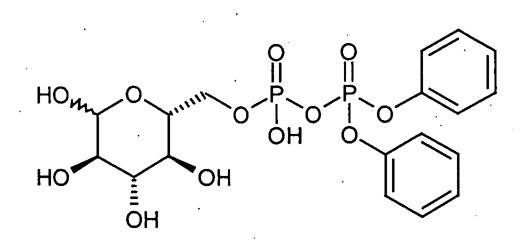

In analogy to A.2 analogs with ¹³C labeled glucose can be prepared.

### Example A.3

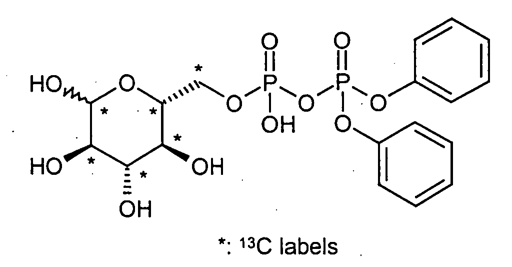

### Example A.4

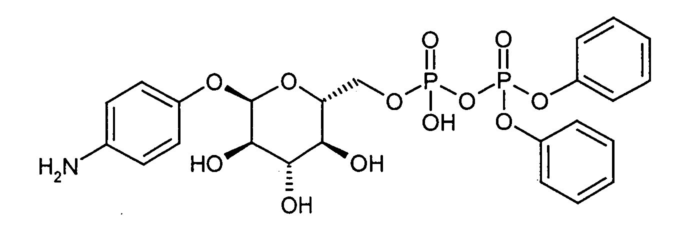

### Example A.5

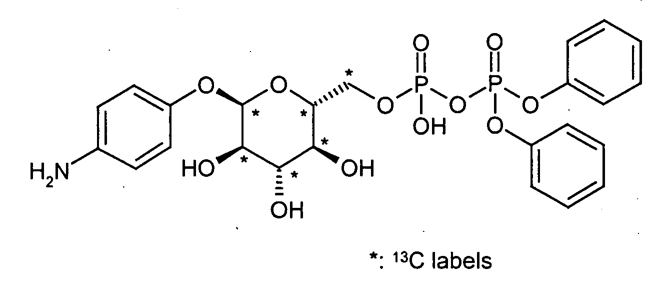

### Example A.6

### Phosphate precursor:

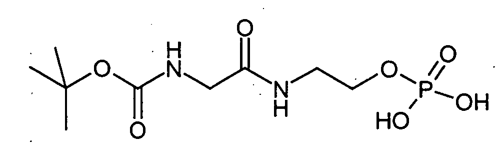

141 mg (1 mmol) O-Phosphoryl-ethanolamine, 356 mg ethyl diisopropylamine and 408 mg (1,5 mol) Boc-glycine-n-hyroxysuccinimide are reacted in methanol for 3 h at room temperature. The solvent is removed in vacuo and the remaining residue is digerated with methanol / dichloromethane and filtrated. The mother liquid is poured into diethyl ether. The precipitate is collected and purified by flash chromatography utilizing acetonitril/water/ glacial acetic acid 5:1:0,2. The relevant fractions are collected, the solvent is removed in vacuo and the remaining residue is digerated with diethyl ether and dried in vacuo. 170 mg (57%) of the desired product are obtained. [TLC: acetonitrile/water/glacial acetic acid 5/1/0,2: R_{f} = 0,2].

Reactive intermediate: (Slight modifications of the general procedure) 30 mg (100 µmol) of this phosphate precursor are suspended in dichloromethane and 48 mg pyridin are added. Subsequently, 35 mg (131 µmol) diphenyl phosphoryl chloride are added and reacted for 3 h. The solvent is removed in vacuo.

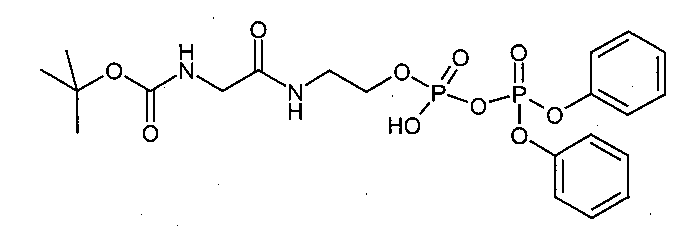

In analogy to example A.6 dipeptide analoges and dipeptide analogs carrying isotopic tags can be prepared.

### Example A.7

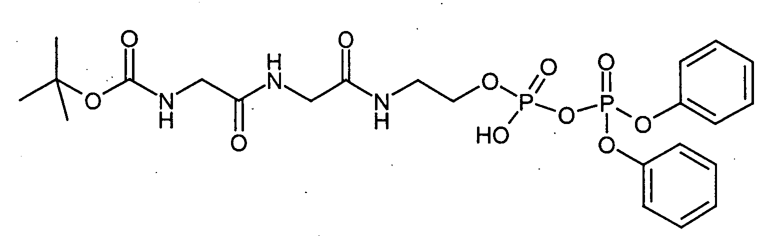

### Example A.8

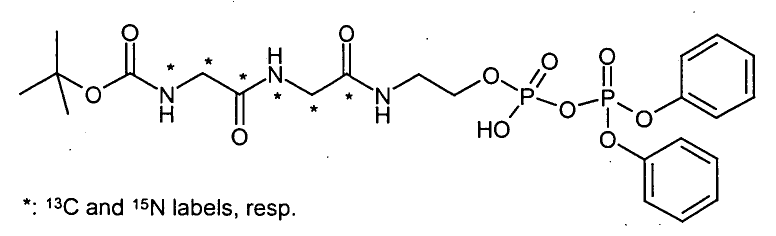

### Example A.9

### Phosphate precursor:

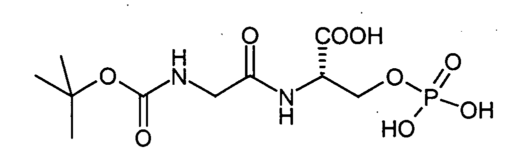

50 mg (270 µmol) O-Phosphoryl-serine, 105 mg ethyl diisopropylamine and 110 mg (405 µmol) Boc-glycine-n-hyroxysuccinimide are reacted in methanol for 3 days at room temperature. The solvent is removed in vacuo and the remaining residue is digerated with methanol / dichloromethane and filtrated. The mother liquid is poured into diethyl ether. The precipitate is collected and purified by flash chromatography utilizing dichloromethane/methanol glacial acetic acid 10:1:0,1. The relevant fractions are collected, the solvent is removed in vacuo and the remaining residue is digerated with diethyl ether and dried in vacuo. 36 mg (39%) of the desired product are obtained. [TLC: acetonitrile/water/glacial acetic acid 5/1/0.2: R_{f}= 0.12].
(ESI-MS: m/e = 341 (M-H)⁻]

### Reactive intermediate:

### Synthesis in analogy to example A.6 (slight modifications of the general procedure).

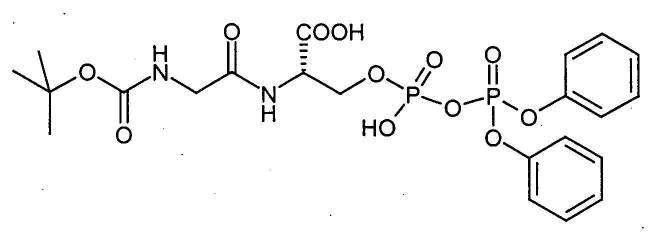

### Example A.10

### Phosphate precursor:

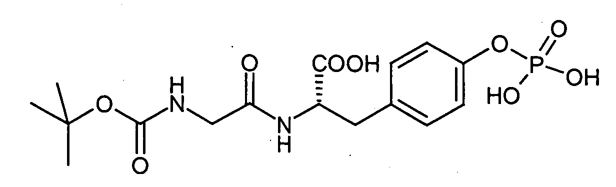

Synthesis in analogy to example A.9 satrting from phospho tyrosine.
Yield: 86%
[TLC: acetonitrile/water/glacial acetic acid 5/1/0.2: R_{f} = 0.27].
(ESI-MS: m/e = 417 (M-H)⁻]

### Reactive intermediate:

Synthesis in analogy to example A.9 (slight modifications of the general procedure).

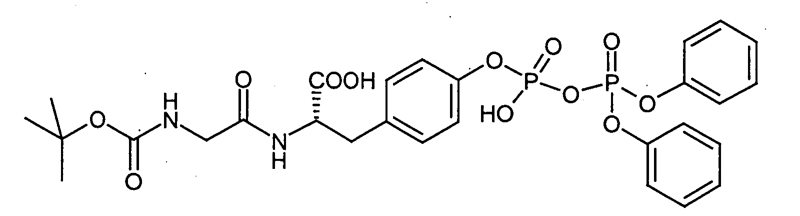

### Example A.11

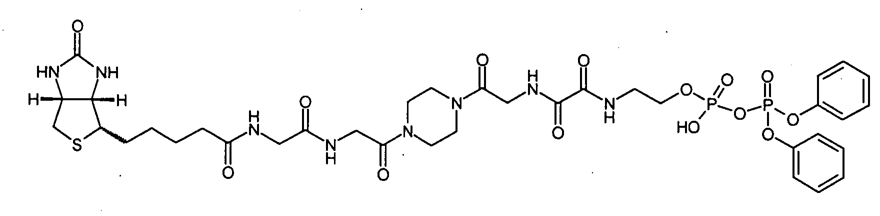

Initially, intermediate D is synthesized as shown in scheme 2 starting from intermediate A following standard procedures well known in peptide chemistry.

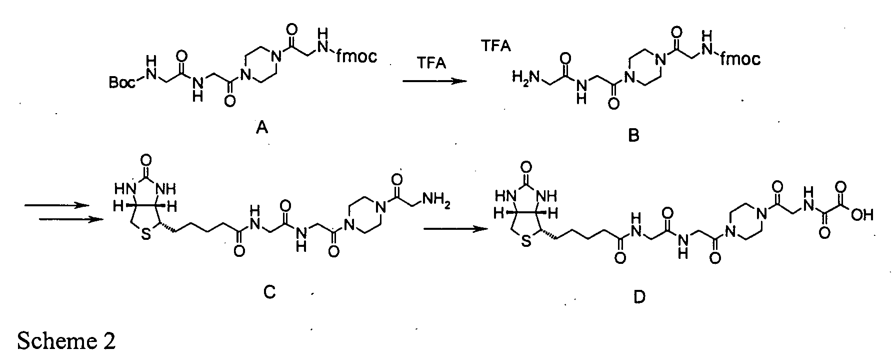

In parallel, the Boc protecting group is cleaved from the phosphate precurser in example A.6 using trifluoro acetic acid.

Subsequently, intermediate D is pre-activated with 1,2 eq. N-(3-dimethylaminopropyl)-N'-ethylcarbodiimide hydrochloride and 1.5 eq. hydroxy-1H-benzotriazole (EDCI / HOBT) and 2 eq. ethyl diisopropylamine in DMF for 1 hour. 1 eq. of the amino deprotected phosphate precurser is added to yield compound A11.

### Example A.12

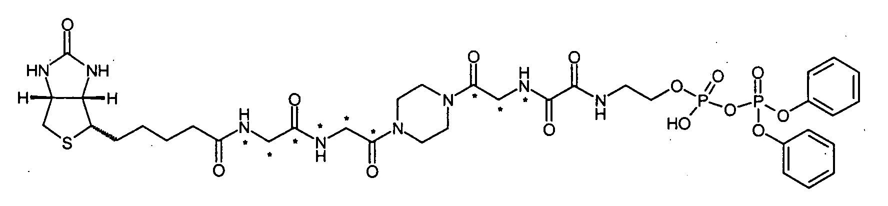

The labelled compound is synthesized in analogy to example 11 starting from commercially available ¹³C and ¹⁵N labelled glycine.

### Example A.13

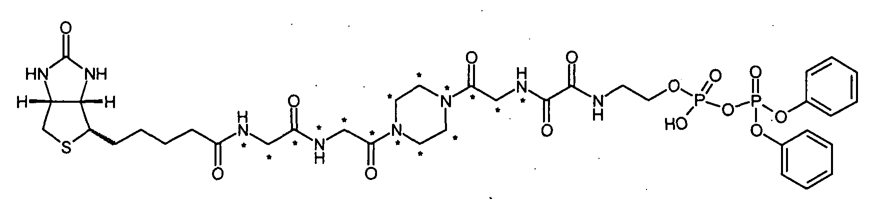

The labelled compound is synthesized in analogy to example 11 starting from commercially available ¹³C and ¹⁵N labelled glycine.

### B. Coupling with phosphorylated amino acids and peptides

### General formula

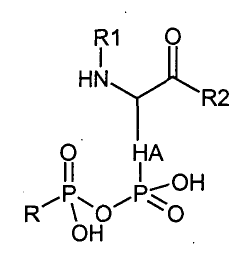

With R1 = H or Fmoc or peptide residue and HA = deprotonated side chain of an hydroxy amino acid and R2 = OH or peptide residue.

### General procedure (unless otherwise stated)

0,207 mmol of the corresponding phosphorylated amino acid or peptide derivative are dissolved in 1 ml methanol with 90,43 µl tri-noctylamine and stirred for 15 min. at RT. The solvent is removed *in vacuo* and the residue is repeatedly treated with DCM which is also removed, in order to eliminate all traces of water.

The product is dried under high vacuum for 1h00 and then, dissolved in 3 ml dry dioxan with 250 µl (3,09 mmol) pyridin. 1,03 mmol of the desired activated pyrophosphate prepared as described in example series A are dissolved in 1 ml dry dioxan and added slowly to the mixture.

After 1h00 of reaction, the solvent is removed *in vacuo* and the residue is purified by *flash* chromatography (CH₃CN/H₂O/AcOH: 10/3/2).

### Example B.1

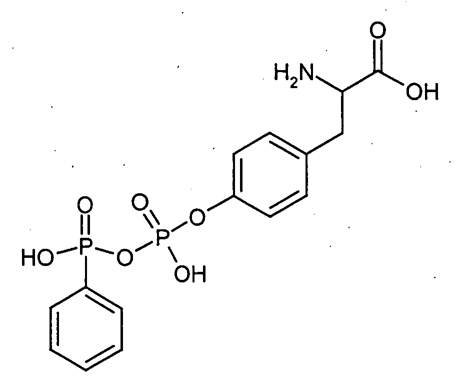

Reactants: phosphotyrosine + 1 eq. A.1
[ESI-MS: m/e = 402 (M+H)⁺].

### Example B.2

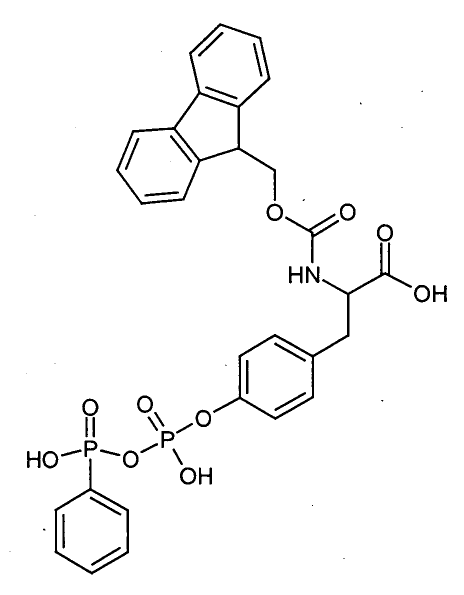

Reactants: Fmoc-phosphotyrosine + A.1*
[ESI-MS (negative ion mode): m/e = 622 (M-H)⁻].
*1 to 99 eq

### Example B.3

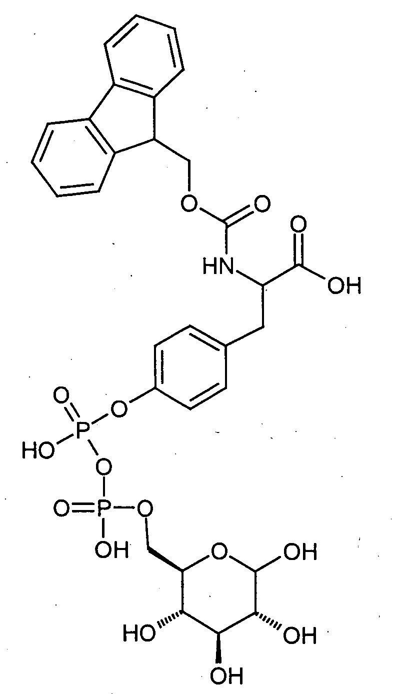

Reactants: Fmoc-phosphotyrosine + A.2
Yield: 58 %
[ESI-MS (negative ion mode): m/e = 724 (M-H)⁻].

### Example B.4

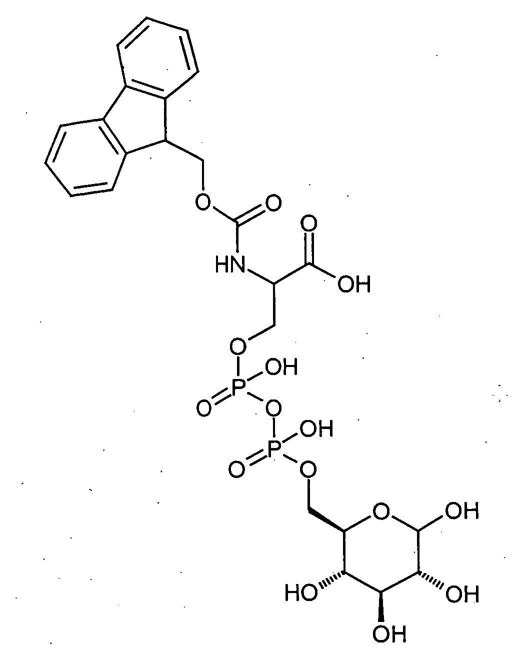

Reactants: Fmoc-phosphoserine + A.2
Yield*: 50 %
[ESI-MS (negative ion mode): m/e = 648 (M-H)⁻].
* reaction time: overnight

### Example B.5

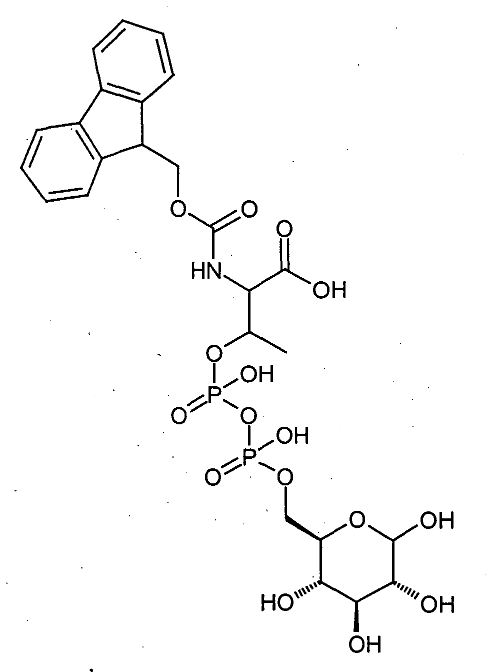

Reactants: Fmoc-phosphothreonine + A.2
Yield*: 14 %
[ESI-MS (negative ion mode): m/e = 662 (M-H)⁻].
* reaction time: overnight

### Example B.6

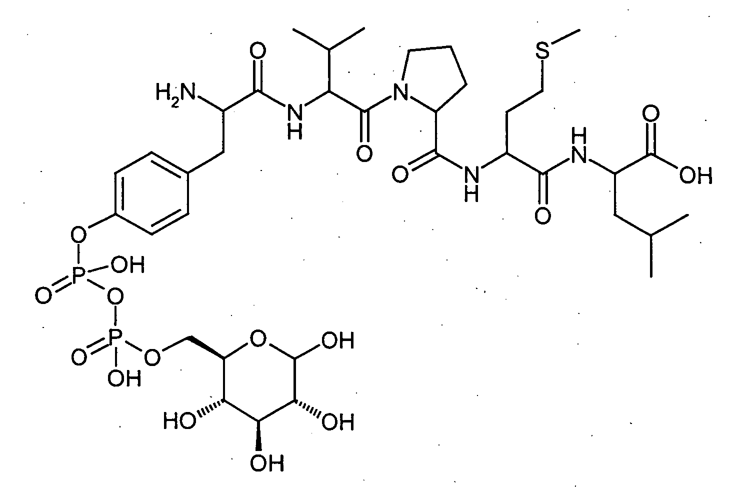

Reactants*: PDGF BETA-RECEPTOR (719-723) (PHOSPHORYLATED) + A.2
[ESI-MS (negative ion mode): m/e = 942 (M-H)⁻].

0,5 mg of PDGF BETA-RECEPTOR (719-723) (PHOSPHORYLATED), solvent: dry DMSO

### Example B.7

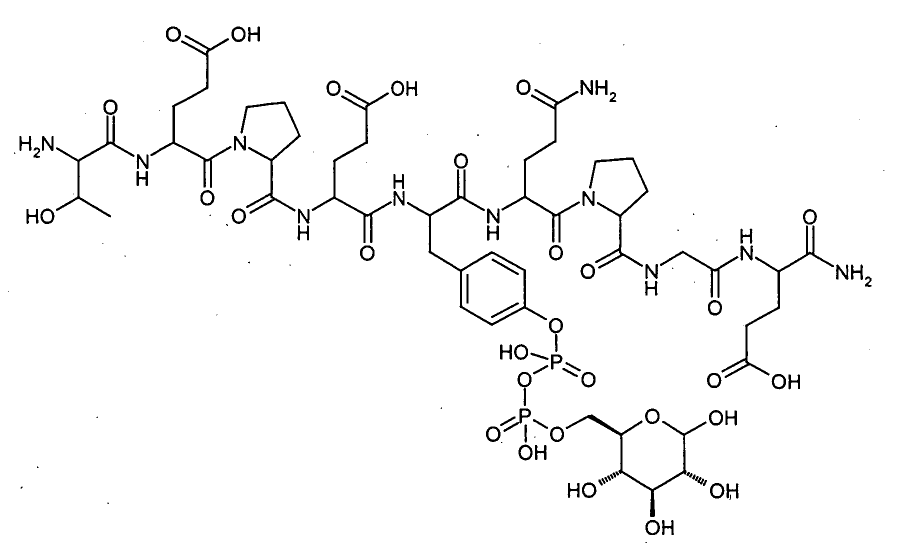

Reactants*: H-THR-GLU-PRO-GLU-TYR(P03H2)-GLN-PRO-GLY-GLU-NH2 + A.2
[ESI-MS (negative ion mode): m/e = 683 (M-2H)²⁻].
* 1 mg of H-THR-GLU-PRO-GLU-TYR(P03H2)-GLN-PRO-GLY-GLU-NH2, solvent: dry DMSO.

### Example B.8

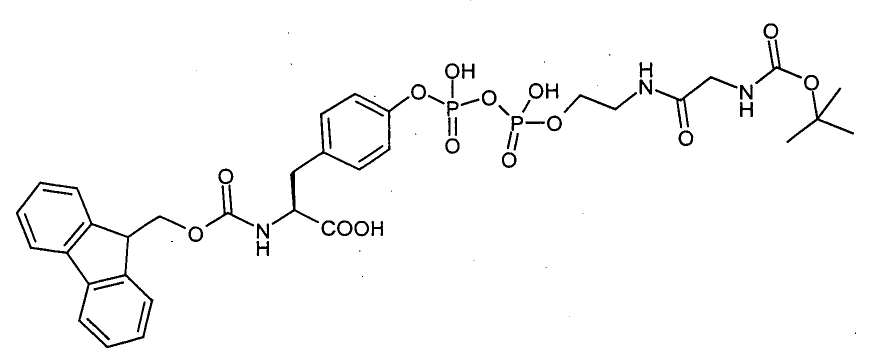

Reactants: Fmoc-phosphotyrosine + A.6
Yield: 79 % [TLC: acetonitrile/water/glacial acetic acid 5/1/0.2: R_{f}= 0.17].
[ESI-MS: m/e = 764 (M+H)⁺].

## Claims

1. Compound of the formula 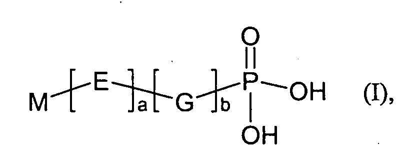 in which
a and beach independently are 0 or 1,
M is selected from the group consisting of
hydrogen,
phenyl,
an amino protecting group, or
a group A-L,
where
A is the residue of an affinity ligand or a solid-phase support and
L is a covalent linker between A and E in the case of a being 1 or between A and G in the case of a being 0,
the linker L having the formula 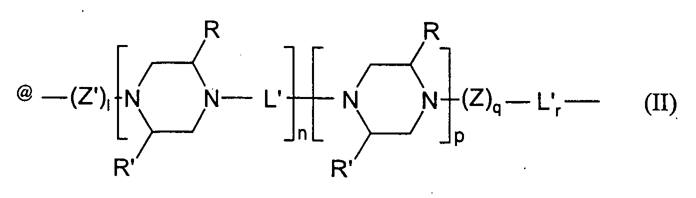 wherein
^{@} denotes the terminus connected to A,
l, n, p, q, r each independently is 0, 1, 2, 3, 4 or 5, provided that their summation 1 + n + p + q + r amounts to at least 1 and not more than 20,
Z' is an amino acid group NH-CHR^{x}-(CH₂)ₓ-CO, where R^{x} is an amino acid side-chain and x is 0, 1, 2, 3, 4 or 5,
L' is a bridge enabling the covalent linkage between two piperazine groups, between two substituted amino groups or between a substituted amino group and a piperazine group,
R, R' are independently selected amino acid side-chains attached to a piperazine ring,
Z is an amino acid residue CO-(CH₂)_{y}-CHR^{y}-NH, where R^{y} is an amino acid side-chain and y is 0, 1, 2, 3, 4 or 5, which is distinct from Z' due to the different orientation of the terminal CO and NH groups,
E is a residue of an amino alcohol moiety of the formula 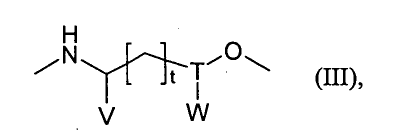 wherein
V is hydrogen, carboxy (-COOH) or an optionally arylsubstituted alkoxycarbonyl,
t is 0, 1, 2, 3, 4 or 5,
W is hydrogen or methyl and
T is CH or p-phenylen
with the proviso that in the case of T being p-phenylen t is 1 and W is hydrogen,
G is a residue of a glucose derivative of the formula 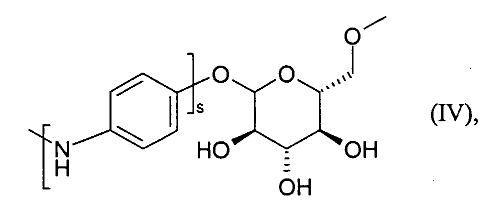 wherein s is 0 or 1 and the configuration at the anomeric carbon atom is α or ß or a mixture of α and ß.
and their salts.

2. Compound of the formula 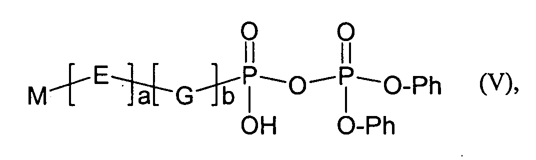 in which
a, b, M, E, G have the same meaning as in claim 1 and
Ph represents phenyl.

3. Compound of the formula 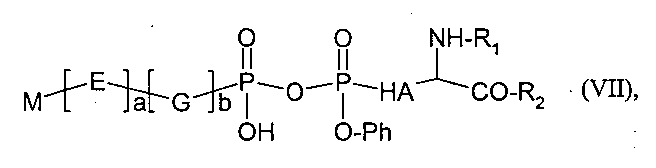 in which
a, b, M, E, G have the same meaning as in claim 1 or 2,
Ph represents phenyl,
HA represents a deprotonated side chain of an hydroxy amino acid,
R₁, R₂ each independently represents a residue of amino and carboxy protecting groups respectively or
are R₁ = H and R₂ = OH or
each independently represents a peptide residue.

4. Compound according to any of the preceding claims, **characterized in that** it is isotope labeled.

5. Compound according to any of the preceding claims, **characterized in that** A represents biotinoyl.

6. Process for the labeling of amino acid phosphates, phosphopeptides or phosphoproteins comprising
(iv) providing one ore more differently isotope labeled compounds of formula (I) according to claim 1,
(v) transforming said one or more compounds to one or more compounds of formula (V) according to claim 2 and
(vi) reacting the one ore more compounds of formula (V) with a sample comprising at least one phosphorylated amino acid, peptide or protein derivative.

7. Use of one or more differently isotope labeled compounds according to any of claims 1 to 5 as reagent for the mass spectrometric analysis of amino acid phosphates, phosphopeptides or phosphoproteins.

8. Use according to the preceding claim for identifying one or more amino acid phosphates, phosphopeptides or phosphoproteins in one or more samples comprising amino acid phosphates, phosphopeptides or phosphoproteins.

9. Use according to one of the two preceding claims for determining the relative expressions levels of one or more amino acid phosphates, phosphopeptides or phosphoproteins in one or more samples comprising amino acid phosphates, phosphopeptides or phosphoproteins.

10. Kit for the mass spectrometric analysis of amino acid phosphates, phosphopeptides or phosphoproteins, comprising as reagents one or more differently isotope labeled compounds according to any of claims 1 to 5.
